# EUROPEAN PATENT APPLICATION

(11) **EP 3 120 881 A1**
(43) Date of publication of application: **25.01.2017**
(21) Application number: 15178046.7
(22) Date of filing: 23.07.2015
(51) Int. Cl.: A61M 1/10

(54) **PULSATILE VENTRICULAR ASSIST DEVICE**

(71) Applicant: Northern Development AS, 0198 Oslo (NO)
(72) Inventor: GRUBYI, Peter, 3300 Hokksund (NO)
(74) Representative: TBK

(57) **Abstract**

A pulsatile ventricular assist device (VAD) has a pumping unit (10) comprising an electric actuator (4) having an movable part (4a) which is movable with a linear stroke with respect to a fixed part (4b), an actuator housing (3) defining an actuator chamber within which the actuator (4) is mounted, and a pumping element in the form of a diaphragm (2) which is coupled to the movable part (4a) of the actuator (4) and hermetically seals an opening of the actuator housing (3). The actuator (4) is operated with a pulsatile blood flow pattern in accordance with the cardiac rhythm of the heart. In a preferred embodiment, the actuator (4) is configured such that one stroke of the movable part (4a) in the direction of the diaphragm (2) takes about the same time as the contraction of the heart.

## Description

The present invention relates to a pulsatile ventricular assist device (pulsatile VAD) designed to support the pumping function of a heart.

Early VADs used pulsatile pumps which alternately sucked blood from the left ventricle of the heart and then forced it out into the aorta, thereby producing a pulsatile blood flow pattern. Pumps of this kind are disclosed in US 4 143 425 A and US 4 360 324 A. These pumps comprise a flexible sac and a reciprocating thrust member acting on the sac.

More recent work concentrated on continuous flow pumps. Pumps of this kind had the advantage of greater simplicity resulting in smaller size and greater reliability. A potential disadvantage of the continuous flow pumps is that a patient in most cases will not have a normal pulse, or that the pulse intensity of the heart will be greatly reduced. For this reason, US 2013/0289336 A1 and US 2014/0323796 A1 propose controlling the operating speed of the continuous flow pump such that a pulsatile blood flow pattern is produced, including time periods of relatively high blood flow rates and time periods of relatively low blood flow rates.

The pulsatile blood flow pattern produced by compressing a flexible sac or controlling the operating speed of a continuous flow pump is still far from being similar to the pulsatile blood flow pattern produced by the natural pumping function of the heart. It is therefore an object of the present invention to provide a pulsatile ventricular assist device (VAD) which is capable of imitating more closely the pulsatile blood flow pattern produced by the natural pumping function of the heart.

According to the present invention, there is provided a pulsatile ventricular assist device (VAD) having a pumping unit, said pumping unit comprising an electric actuator having a movable part which is movable with a linear stroke with respect to a fixed part, an actuator housing defining an actuator chamber within which the actuator is mounted, and a pumping element in the form of a diaphragm which is coupled to the movable part of the actuator and hermetically seals an opening of the actuator housing.

In the context of the present specification, a diaphragm is understood as a sheet of a semi-flexible material which is anchored at its periphery. Preferred examples of the diaphragm material include silicon rubber, segmented polyurethane (SPU), elastomeric polytetrafluoroethylene (PTFE), ethylene propylene diene monomer rubber (EPDM) etc. The diaphragm may have a two-layered structure, the layer coupled to the movable part being the above-mentioned diaphragm material and the other layer being a biocompatible material such as bovine pericardium.

As indicated above, the pumping unit is structured such that the diaphragm hermetically seals the opening of the actuator housing. In this way, the diaphragm hermetically seals the actuator chamber from an area where the blood flows.

The opening of the actuator housing may have a diameter from 25 mm to 55 mm, preferably from 30 mm to 50 mm, more preferably in the range between 35 mm and 45 mm. The actuator housing may have a flange at the opening and the diaphragm may be anchored to the flange.

The diaphragm may be folded towards the inside of the accommodating chamber at an intermediate portion of the diaphragm between the periphery of the diaphragm where the diaphragm is anchored and an inner portion of the diaphragm where the movable part of the actuator is coupled to the diaphragm. The folding is preferably of a size allowing the diaphragm to be moved without stretching. If the material of the diaphragm has sufficient flex fatigue resistance, a flat diaphragm without folding may be used instead. For example, BioSpan™ SPU, manufactured by DSM Biomedical Inc., has excellent flex life.

In one preferred embodiment of the present invention, the pumping unit further comprises a pumping chamber housing defining a pumping chamber having an inlet for connection with the left or right ventricle of the heart and an outlet for connection with the aorta, subclavian artery or the pulmonary artery, respectively. The inlet is provided with a check valve for preventing a backflow of blood to the heart. The outlet may also be provided with check valve although this is optional. Examples of the check valve include a silicon check valve, a carbon check valve, etc. The pumping chamber housing has an opening which faces the opening of the actuator housing and is preferably of about the same size as the opening of actuator housing. The pumping chamber housing and the actuator housing are coupled to each other by flanges, for example, with the diaphragm being sandwiched between the flanges.

In another preferred embodiment of the present invention, the pumping unit does not comprise the above-mentioned pumping chamber housing, but is configured to be directly attached to the heart. The pumping unit may further comprise an outer casing which encases the actuator housing and is to be used for attaching the pumping unit directly to the heart. In this alternative embodiment, the pumping unit uses the ventricle of the heart as a pumping chamber and the heart valves (i.e., the atrioventricular valve at the inlet of the ventricle and the semilunar valve at the outlet of the ventricle) as check valves. At least the atrioventricular valve must be functional for enabling the pumping unit to work properly.

Examples of the electric actuator are a voice coil actuator, a piezoelectric actuator, and a linear motor. These kinds of actuators provide a highly accurate linear stroke under high acceleration. It is preferred that one stroke of the movable part in the direction of the diaphragm requires not more than 0.3 s, preferably not more than 0.2 s, or not more than 0.1 s. The shorter the upstroke time is, the higher the flow rate of the pumping unit tends to be. It is most preferred that the stroke takes about the same time or even less time than the contraction of the heart, also called ventricular systole, during which blood is forced into the aorta and pulmonary artery. The contraction lasts about 0.25 s. Among the above-mentioned examples of the electric actuator, the voice coil actuator and the piezoelectric actuator tend to have a size small enough to realize an implantable VAD.

It is preferred that a return stroke of the movable part of the actuator in the direction opposite to the direction of the diaphragm requires more time than the stroke in the direction of the diaphragm. The return stroke has no effect on the flow rate of the pumping unit. If the return stroke takes more time, acceleration and electric power consumption will be less.

The displacement of the movable part depends on the diameter of the diaphragm and is preferred to be in the range from 5 mm to 25 mm, more preferably in the range between 10 mm and 20 mm. At present, a displacement of 10 mm is considered as being sufficient for pumping over 35 ml of blood during each stroke of the actuator or 2.2 l/min, and a displacement of 25 mm is considered as being sufficient for pumping over 50 ml of blood or 3.2 l/min at 60 beats per minute, over 4.5 l/min at 90 beats per minute and over 6 l/min at 120 beats per min. The smaller the displacement is, the smaller the pumping unit can be and the less electric power is consumed by the actuator.

Preferably, the actuator is provided with a preloading member between the movable part and the fixed part that applies a preload to the moveable part in the direction of the diaphragm. The preload reduces the load on the diaphragm at diastolic peak pressure, and the pressure difference to be generated by the actuator during systole is reduced.

The VAD may comprise a control unit that is configured to cause the actuator to operate with a pulsatile blood flow pattern in accordance with the cardiac rhythm of the heart. For this purpose, the control unit may use, for example, the input signal from a pacemaker, an accelerometer or a pressure sensor. In one mode of control, the control unit is configured to change the time period between two subsequent strokes of the actuator in accordance with the pulse rate. In another mode of control, if required, the control unit is configured to change both the time period between two subsequent strokes of the actuator and the displacement of the movable part in accordance with the pulse rate.

According to the present invention, there is provided a VAD which is capable of imitating more closely the pulsatile blood flow pattern produced by the natural pumping function of the heart. In particular, the pumping unit of the VAD is capable of producing a pressure pulse with a peak amplitude of 150 mbar (120 mmHg systolic pressure) within less than 0.2 s and pumping 35 to 50 ml of blood during each stroke or 2.2 to 3.2 l/min at 60 beats per minute, over 4.5 l/min at 90 beats per minute and over 6 l/min at 120 beats per min.

In the following, the invention will be described with reference to the drawings by way of preferred embodiments and test results.
Fig. 1 is a cross-sectional view showing a pumping unit of a VAD according to a first embodiment of the present invention.
Fig. 2 is a cross-sectional view showing a pumping unit of a VAD according to a second embodiment of the present invention.
Fig. 3 is a schematic diagram showing the pumping unit of the VAD according to the second embodiment, which assists the left ventricle of a human heart.
Fig. 4 is a cross-sectional view showing a pumping unit of a VAD according to a third embodiment of the present invention.
Fig. 5 is a graph showing the pressure measured during one cycle of test no. 4.
Fig. 6 shows the setup used in the tests.

### First Embodiment

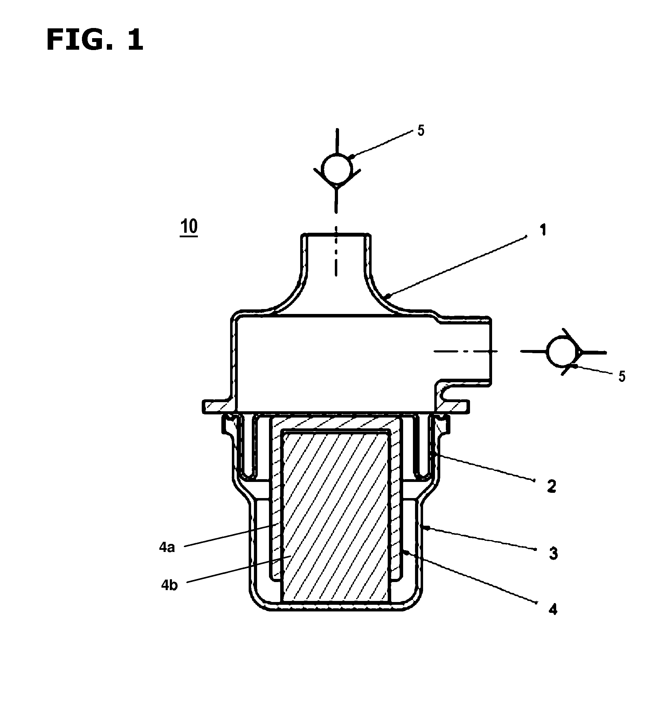

Fig. 1 shows a pumping unit 10 of a VAD according to a first embodiment of the present invention, which is to be implanted to a patient. In addition to the pumping unit 10, the VAD comprises a control unit and battery pack which are to be externally worn by the patient, an electrical connection cable for connecting the pumping unit 10 with the control unit and the battery pack, and an inlet conduit for connecting the pumping unit 10 with the left or right ventricle, or left or right atrium of the heart and an outlet conduit for connecting the pumping unit 10 with the aorta, subclavian artery or the pulmonary artery, respectively.

As shown in Fig. 1, the pumping unit 10 comprises: an actuator 4 having a movable part 4a which is movable with a linear stroke with respect to a fixed part 4b; an actuator housing 3 defining an actuator chamber within which the actuator 4 is mounted; and a diaphragm 2 which is coupled to the movable part of the actuator 4 and hermetically seals an opening of the actuator housing 3. The pumping unit 10 further comprises a pumping chamber housing 1 which defines a pumping chamber and has an inlet for connection with the left or right ventricle of the heart and an outlet for connection with the aorta or the pulmonary artery, respectively.

The inlet and outlet of the pumping chamber housing 1 are each provided with a check valve 5 for preventing a backflow of blood to the heart. The check valve 5 at the outlet of the pumping chamber housing 1 may be omitted.

The pumping chamber housing 1 has an opening which faces the opening of the actuator housing 3. The diameter of the opening is 40 mm and is about the same size as the opening of the actuator housing 4. The pumping chamber housing 1 and the actuator housing 4 are coupled to each other by flanges. The diaphragm 2 is anchored to a flange of the actuator housing 3 and hermetically seals the actuator chamber in the actuator housing 4 from the pumping chamber in the pumping chamber housing 1 where the blood flows.

The diaphragm 2 has a two-layered structure including an inner layer which is coupled to the movable part 4a of the actuator 4 and an outer layer which faces the pumping chamber in the pumping chamber housing 1. The inner layer is made of semi-flexible polyurethane and the outer layer may be made of a biocompatible membrane, like bovine pericardium.

The diaphragm 2 is folded towards the inside of the accommodating chamber at an intermediate portion of the diaphragm 2 between the periphery of the diaphragm 2 where the diaphragm is sandwiched between the pumping chamber housing 1 and the actuator housing 4 and an inner portion of the diaphragm 2 where the movable part 4a of the actuator 3 acts on the diaphragm 2. The folding is of a size allowing the diaphragm 2 to be moved without stretching.

In the first embodiment, the actuator 4 is a voice coil actuator having a coil assembly as the movable part 4a and a permanent magnet assembly as the fixed part 4b. The voice coil actuator 4 is configured such that the movable part 4a can be displaced in a range from 10 mm to 25 mm in the direction of the diaphragm 2 and one stroke of the movable part 4a in the direction of the diaphragm 2 requires not more than 0.2 s (i.e., about the same time as the contraction of the heart, also called ventricular systole, during which blood is forced into the aorta, subclavian artery or pulmonary artery). Although the displacement seems to be small at a first glance, the displacement is sufficient for pumping over 35 to 50 ml of blood during each stroke of the actuator or 2.2 to 3.2 l/min at 60 beats per minute.

The control unit of the VAD is configured to cause the actuator 4 to operate in accordance with the cardiac rhythm of the heart. For this purpose, the control unit uses the input signal from a pacemaker, accelerometer or pressure sensor and changes the time period between two subsequent strokes of the actuator 4 in accordance with the pulse rate. If the actuator 4 is a variable displacement actuator, the control unit may change not only the time period between two subsequent strokes of the actuator 4 in accordance with the pulse rate, but also the displacement of the movable part 4a of the actuator 4.

### Second Embodiment

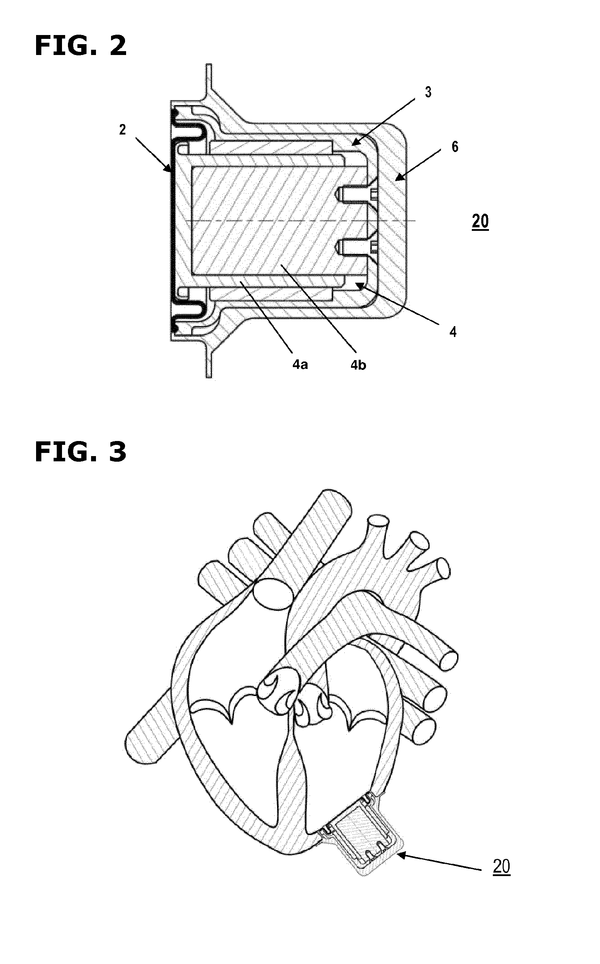

Fig. 2 shows a pumping unit 20 of a VAD according to a second embodiment of the present invention, which is to be implanted to a patient. In addition to the pumping unit 20, the VAD comprises a control unit and battery pack which are to be externally worn by the patient, and an electrical connection cable for connecting the pumping unit 20 with the control unit and the battery pack.

As shown in Fig. 2, the pumping unit 20 comprises: an actuator 4 having a movable part 4a which is movable with a linear stroke with respect to a fixed part 4b; an actuator housing 3 defining an actuator chamber within which the actuator 4 is mounted; and a diaphragm 2 which is coupled to the movable part 4a of the actuator 4 and hermetically seals an opening of the actuator housing 3. An outer casing 6 encases the actuator housing 3 and is used for attaching the pumping unit 20 directly to the heart.

The structure and arrangement of the actuator 4, the actuator housing 3, and the diaphragm 2 are the same as in the first embodiment so that further explanation is omitted.

Fig. 3 shows the pumping unit 20 in a state where it is implanted to the patient. The pumping unit 20 is inserted through a surgical cut in the heart's wall with the diaphragm 2 forming part of the left ventricle. The outer casing 6 has a flange ring which contacts the outer surface of the heart's wall so that the diaphragm 2 is about flush with the inner surface of the heart's wall and does not obstruct the blood flow within the left ventricle. The pumping unit 20 is preferentially held within the pericardial sac.

During the operation of the VAD, the pumping unit 20 uses the left ventricle of the heart as a pumping chamber and the heart valves, that is the atrioventricular (mitral) valve at the inlet of the left ventricle and the semilunar valve at the outlet, as check valves. Unlike the first embodiment, the second embodiment does not need the pumping chamber housing 1 to produce the pulsatile blood flow pattern. Similar to the first embodiment, at least the inlet valve, that is the atrioventricular (mitral) valve, must be functional for enabling the pumping unit 20 to work properly and avoid backflow of blood to the atrium of the heart.

It goes without saying that the pumping unit 20 of the VAD according to the second embodiment can also be connected to the right ventricle of the heart.

### Third Embodiment

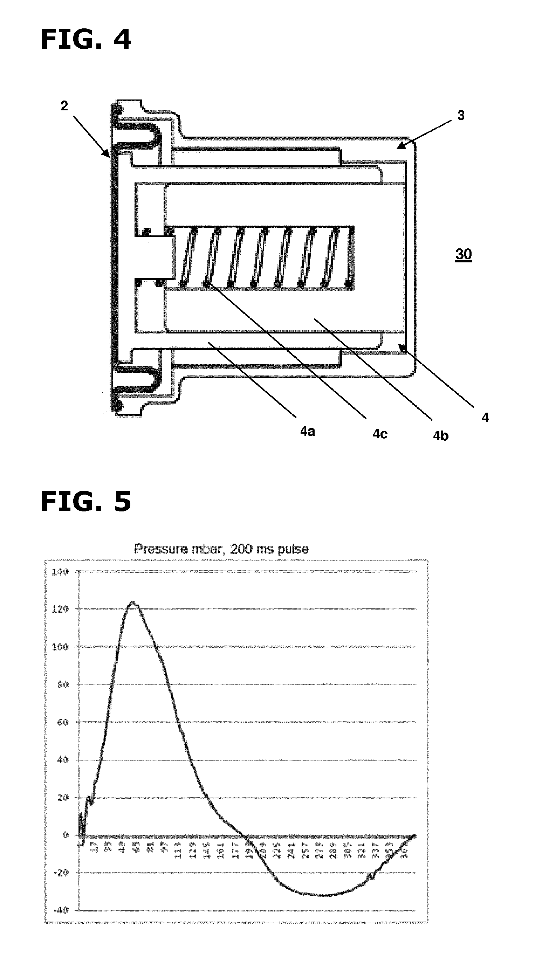

Fig. 4 shows a pumping unit 30 of a VAD according to a third embodiment of the present invention.

As shown in Fig. 2, the pumping unit 30 comprises: an actuator 4 having a movable part 4a which is movable with a linear stroke with respect to a fixed part 4b; an actuator housing 3 defining an actuator chamber within which the actuator 4 is mounted; and a diaphragm 2 which is coupled to the movable part 4a of the actuator 4. The actuator 4 is provided with a preloading member 4c between the movable part 4a and the fixed part 4b that applies a preload to the moveable part 4a in the direction of the diaphragm 2.

Except for the preloading member 4c, the structure and arrangement of the actuator 4, the actuator housing 3, and the diaphragm 2 are the same as in the first embodiment and the second embodiment so that further explanation is omitted.

The preloading member 4c may include a magnetic or mechanical spring, a hydraulic accumulator or any adjustable elastic element which is capable of applying a set preload to the moveable part 4a of the actuator 4 in the direction of the diaphragm 2. Preferably, the preload is set to be the same as the diastolic peak pressure acting on the diaphragm 2 during operation of the VAD. The reason is given below.

When the left ventricle is refilled with blood during diastole in a 24 year old male patient (reference patient), the diastolic peak pressure in the left ventricle is 80 mmHg (100 mbar). When the left ventricle contracts during systole, the systolic peak pressure is 120 mmHg (150 mbar). If the VAD according to the present invention is operated in such a way as to generate a systolic pressure of 120 mmHg synchronously with the left ventricle by pumping blood into the aorta, it is preferable to neutralize the diastolic pressure acting on the diaphragm 2 during diastole. For example, if the diaphragm has a diameter of Ø 50 mm, the pressure acting on the diaphragm 2 will be 19.6 N at 80 mmHg. Therefore, the preload of the preloading member 4c should be set to 19.6 N in order to neutralize the load. This will reduce the load on the diaphragm 2 at diastolic peak pressure, and the pressure difference to be generated by the actuator 4 during systole will be 40 mmHg (i.e., 120 mmHg of systolic peak pressure - 80 mmHg of the preload).

Depending on the mode of use, the pumping unit 30 according to the third embodiment further comprises the pumping chamber housing 1 described in the first embodiment, or the outer casing 6 described in the second embodiment.

### Modifications

It is obvious that modifications and/or additions can be made to the VADs described heretofore as long as these remain within the field and scope of the invention.

For example, the VAD can have an internal controller and battery without the cable to an external controller and battery pack. In this case the battery can be charged via a wireless charger.

According to the first embodiment, the VAD is to be permanently placed inside the patient's body with its inlet connected to a ventricle and its outlet connected to the aorta, the subclavian artery or the pulmonary artery. Alternatively, the invention may be used as a temporary assist device. In this case, the VAD may be placed outside the body, functioning as an external ventricle assist device being connected with its inlet or in communication with a ventricle or atrium applied by a percutaneously or minimally invasive procedure or during open surgery, and its outlet connected or in communication with the aorta, the subclavian artery or the pulmonary artery applied by a percutaneously or minimally invasive procedure or open surgery.

Further, the folded diaphragm used in the above embodiments can be replaced by a flat diaphragm without folding which is made of BioSpan™ SPU, manufactured by DSM Biomedical Inc., or the like.

Still further, the voice coil actuator used in the above embodiments can be replaced by a piezoelectric actuator or a linear motor.

### Test Results

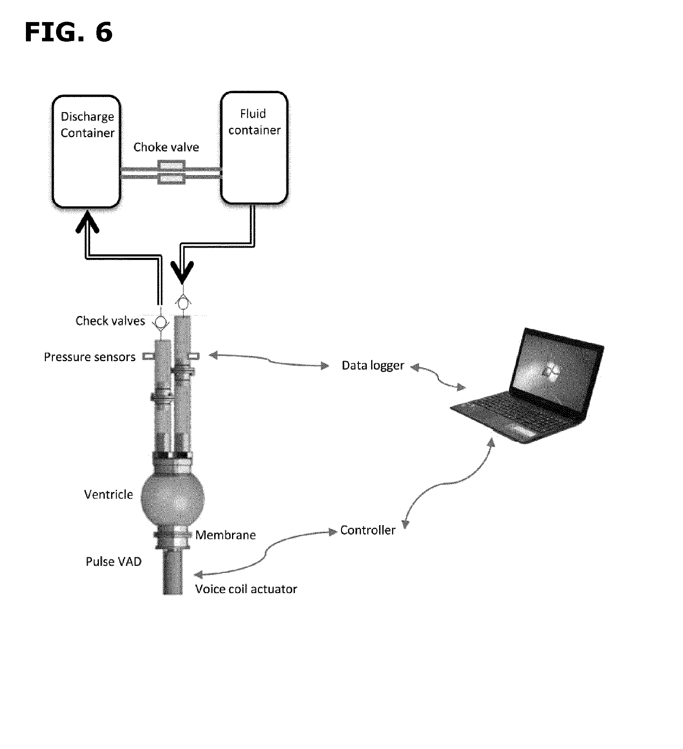

Using the test setup shown in Fig. 6, feasibility of the pumping unit of the pulsatile VAD was tested, in particular whether it meets a flow rate in the range between 2.2 and 3.2 liters per minute together with a pressure which is comparable with the cardiac output peak pressure of approximately 120 mbar.

The pumping unit was directly connected to a hollow body resembling the left ventricle of a heart. For the actuator of the pumping unit a voice coil actuator "CBL35-025-75-1F3" was used. For the pressure sensor a "HKM-375m-1.7bara" was used. For the data logger a "HBM EspressoDAQ DQ-430" was used. "SMAC" control software and "Catman Easy" were run on a laptop computer to control the actuator and log the data.

The variable parameters were:
- amplitude ptp [mm] (ptp: peak-to-peak value of the linear actuator motion),
- curve shape: sine or pulse shape,
- number of beats per minute.

The pulse shape was a self-defined curve which had a specific upstroke and downstroke time and was applied with at various frequencies.

The following parameters were logged in real time by the voice coil actuator software and data logger for further analysis:
- current used by linear motor [A],
- programmed and actual displacement [mm],
- pressure at discharge from pumping unit [bar],
- force [N].

Furthermore, the static pressure in the fluid container of the test setup was kept at approximately 40 mbar while the pressure in the discharge container was kept at 80 mbar. The discharge peak pressure was kept at approximately 120 mbar.

The flow rate was measured by weighing the outflow captured in the discharge container of the test setup during a certain period of time. The available flow meters could not be used as these were designed for continuous flow and did not measure pulsating flow accurately.

The test results are shown in Table 1 below.

**[Table 1]**

| Test No. | Membrane/ Curve shape | Beats [min⁻¹] | Displacement [mm] | Flow rate [l/min] | Source |
|---|---|---|---|---|---|
| 1 | Membrane Ø 50 mm, Sine | 60 | 10 | 1.17 | Calculation |
| 2 | Sine | 60 | 10 | 1.14 | Experimental |
| 3 | Pulse 300 ms up 600 ms down | 60 | 10 | 1.34 | Experimental |
| 4 | Pulse 200 ms up 400 ms down | 60 | 10 | 1.76 | Experimental |
| 5 | Pulse 150 ms up 300 ms down | 60 | 10 | 2.24 | Experimental |
| 6 | Pulse 200 ms up 300 ms down | 60 | 25 | 3.26 | Experimental |

Fig. 5 is a graph showing the pressure measured during one cycle of test no. 4.

As shown in Table 1, the sine curve produced a slightly lower flow rate than calculated while the pulse shaped waves produced up to twice as much flow in otherwise comparable circumstances. The only parameter changed is the motion profile, i.e. time. It is this effect that makes the pumping unit so efficient. The VAD according to the present invention can therefore be more effective compared to, for example, conventional piston or rotary pump devices which use a sine shaped actuation movement, constant or intermittent change in rotation speed.

## Claims

1. Pulsatile ventricular assist device having a pumping unit (10, 20, 30), said pumping unit (10, 20, 30) comprising an electric actuator (4) having a movable part (4a) which is movable with a linear stroke with respect to a fixed part (4b), an actuator housing (3) defining an actuator chamber within which the actuator (4) is mounted, and a pumping element in the form of a diaphragm (2) which is coupled to the movable part (4a) of the actuator (4) and hermetically seals an opening of the actuator housing (3).

2. Pulsatile ventricular assist device according to claim 1, wherein the opening has a diameter from 25 mm to 55 mm.

3. Pulsatile ventricular assist device according to claim 1 or 2, wherein the actuator housing (3) has a flange at the opening and the diaphragm (2) is anchored to the flange.

4. Pulsatile ventricular assist device according to any one of claims 1 to 3, wherein the pumping unit (10) further comprises a pumping chamber housing (1) defining a pumping chamber having an inlet for connection with the left or right ventricle of the heart, or left or right atrium and an outlet for connection with the aorta, subclavian artery or the pulmonary artery, respectively,
wherein the inlet is provided with a check valve (5) for preventing a backflow of blood to the heart.

5. Pulsatile ventricular assist device according to any one of claims 1 to 3, wherein the pumping unit (20) does not comprise a pumping chamber housing, but is configured to be directly attached to the heart.

6. Pulsatile ventricular assist device according to claim 5, wherein the pumping unit (20) further comprises an outer casing (6) which encases the actuator housing (3) and is to be used for attaching the pumping unit (20) directly to the heart.

7. Pulsatile ventricular assist device according to any preceding claim, wherein the actuator (4) is configured such that one stroke of the movable part (4a) in the direction of the diaphragm (2) requires not more than 0.3 s, preferably not more than 0.2 s, or not more than 0.1 s.

8. Pulsatile ventricular assist device according to any preceding claim, wherein the actuator (4) is configured such that a return stroke of the movable part (4a) in the direction opposite to the direction of the diaphragm (2) requires more time than the stroke in the direction of the diaphragm (2).

9. Pulsatile ventricular assist device according to any preceding claim, wherein the actuator (4) is configured such that the displacement of the movable part (4a) is in the range from 5 mm to 25 mm, more preferably in the range between 10 mm and 20 mm.

10. Pulsatile ventricular assist device according to any preceding claim, wherein the actuator (4) is provided with a preloading member (4c) between the movable part (4a) and the fixed part (4b) that applies a preload to the moveable part (4a) in the direction of the diaphragm (2).

11. Pulsatile ventricular assist device according to any preceding claim, further comprising a control unit that is configured to cause the actuator (4) to operate with a pulsatile blood flow pattern in accordance with the cardiac rhythm of the heart.

12. Pulsatile ventricular assist device according to claim 11, wherein the control unit is configured to change the time period between two subsequent strokes of the actuator (4) in accordance with the pulse rate.

13. Pulsatile ventricular assist device according to claim 11, wherein the control unit is configured to change the time period between two subsequent strokes of the actuator (4) and the displacement of the movable part (4a) of the actuator (4) in accordance with the pulse rate.
